# EUROPEAN PATENT APPLICATION

(11) **EP 4 751 578 A1**
(43) Date of publication of application: **03.06.2026**
(21) Application number: 25165455.4
(22) Date of filing: 21.03.2025
(51) Int. Cl.: A23L 29/231, A23L 29/30, A61K 47/26, A61K 47/36

(54) **PECTIN GEL AND PREPARATION METHOD THEREOF**

(30) Priority: 29.11.2024 CN 202411731292
(71) Applicant: Sirio Pharma (Guangdong) Co., Ltd., Shantou Guangdong 515000 (CN)
(72) Inventor: Yu, Ziping, Shantou, Guangdong, 515000 (CN); Cui, Shaohua, Shantou, Guangdong, 515000 (CN); Huang, Weizhao, Shantou, Guangdong, 515000 (CN)
(74) Representative: Petraz, Gilberto Luigi

(57) **Abstract**

Provided in the present application is a pectin gel and a preparation method thereof, the pectin gel including a pectin and a fat-soluble substance, in which a weight average molar weight of the pectin is in a range of 5.0× 10^4 to 6.0× 10^5 Da, a polymer dispersion index thereof is in a range of 1 to 6; in the structural domains of the pectin, HG > 30 mol% and RG-1 ≤ 35 mol%; HG is homogalacturonan and RG-1 is rhamnogalacturonan type I. The pectin gel has high emulsion stability before forming, so the pectin gel after forming has good texture stability, and the amount of water and oil separation is low, and the taste of the pectin gel is good.

## Description

### TECHNICAL FIELD

The present application relates to the technical field of food and medicine and, particularly, relates to a pectin gel and a preparation method thereof.

### BACKGROUND

Pectin is an acidic heteropolysaccharide made of D-galacturonic acid linked by α-1.4-glycosidic bond, which is widely found in the cell walls of higher plants, accounting for about 30%-35%, and it is a natural, green, and healthy natural polymer. Pectin has excellent properties in such as hemostasis, antimicrobial, hypolipidemic, and detoxification, which allows it to be widely used in many fields such as nutraceutical products, food additives, cosmetics, and pharmaceuticals. Pectin regulates the osmotic pressure and pH value of cells, and chewing gel with a certain hardness and modulus may be formed by adding a certain amount of water.

In recent years, pectin has been widely used as a food additive for the preparation of gels containing fat-soluble substances in the food, pharmaceutical, and nutraceutical fields. In the relevant research process, when the oil carrying capacity in the pectin gel (such as OMEGA-3, derived from algae oil, fish oil, and flaxseed oil) is ≥ 10%, it is found that the gel performance and emulsification performance of the pectin gel system are poorly compatible, or the particle size of the oil droplets is great, the particle size distribution of the fat-soluble components is laterally shifted, or the gel structure is loose, and the water locking and oil holding capacity are insufficient, which is "oil separation and water out". Further, the higher the oil carrying capacity, the worse the appearance stability of the product.

At present, there are some relevant studies on pectin gels containing fat-soluble substances, but not many. For example, in CN111803439A, the separation of water and oil and the texture are improved through the combination of stabilizers. However, based on the need for oil holding stability, the introduced stabilizers are mainly saturated/hydrogenated fatty acids, which have the risk of introducing trans-fatty acids. Consumption by humans by mouth may affect digestion and metabolism. For example, in CN114009566B, sugar, as the main vegetable gel matrix component, is replaced by sugar substitute, and the texture of vegetable gel is maintained by the type combination and ratio of sugar substitute, and the oil load is ≥ 10%, which has certain limitations. It is prone to precipitation of oil and water, affecting the appearance and edible process experience, affecting the stability of the content of fat-soluble substances in the pectin gel, and further affecting the digestion and absorption of the human body. Especially for vegetable gels with an oil carrying capacity ≥ 10%, the existence of the water-oil interface of the system requires the system premise of compatibility and stabilization of emulsion droplets first, afterward the texture is rendered, and the texture improvement is further achieved. However, the patent fails to cover this technical scope.

In view of this, how to further improve the stability of pectin gel emulsions containing fat-soluble substances to improve the texture stability of pectin gels and reduce precipitation of water and oil is of great significance to further improve the performance of pectin gel products.

### SUMMARY

In order to solve the problems and shortcomings existing in the prior art, provided in the present application is a pectin gel and a preparation method thereof. The emulsion stability of the pectin gel before forming is high, so the pectin gel after forming has relatively good texture stability, and the amount of precipitation of water and oil is low, and the taste of the pectin gel is relatively good.

According to a first aspect of the present application, provided is a pectin gel, including a pectin and a fat-soluble substance. A weight average molar weight of the pectin is in a range of 5.0×10^4 to 6.0×10^5 Da, and a polymer dispersion index thereof is in a range of 1 to 6. In the structural domains of the pectin, HG > 30 mol% and RG-1 ≤ 35 mol%; HG is homogalacturonan and RG-1 is rhamnogalacturonan type I.

When pectin is paired with a fat-soluble substance, the fat-soluble substance can fill the voids in the pectin gel network, interacting with the pectin molecules to enhance the stability of the gel. However, there may be mutual constraints between them, which may affect the stability of the pectin gel texture, the precipitation of water and oil, and the taste of the pectin gel. Especially in pectin gels containing high content of fat-soluble substances, both high sugar and low sugar pectin gels are prone to precipitation of water and oil, which in turn affects the texture stability and taste of pectin gels. Therefore, it is of great significance for the further development of pectin gels if the texture stability of pectin gels containing high levels of fat-soluble substances is further improved, the precipitation of water and oil is further reduced, and the taste thereof is further improved.

Thus, by controlling the weight average molar weight, polymer dispersion index (PDI), and HG, RG-1 of the pectin, the stability of the mixture material solution (emulsion) before forming is effectively improved, so the stability of the pectin gel texture after forming is improved, the precipitation of water and oil of the pectin gel is effectively reduced, the storage stability of the pectin gel is greatly improved, and the taste of the pectin gel is improved.

Specifically, firstly, with respect to the weight average molar weight of pectin, if it is too low, the interaction between chains is weak, and there is not enough chain length to build a stable three-dimensional gel network structure. Even if a gel is formed, its strength is very weak. Due to the short molecular chains, there are few connection points formed between molecules, and the gel network is not tight and strong enough. In the end, the gel elasticity of pectin formation is insufficient, the texture stability is poor, and the gel network is not tight and strong enough. It will also lead to problems such as prone to precipitation of water and oil. If it is too great, the solubility of the pectin is poor, it is not easy to form a uniform and stable system with other materials, especially the emulsion system is more susceptible, and if the conditions are not properly controlled, it may not be possible to form a gel or form a non-uniform gel. If a gel is formed, it will also lead to excessive strength, excessive texture, affect the taste, lack of elasticity and flexibility, easy to break when subjected to external forces, and also affect the storage stability of the pectin gel.

Secondly, with respect to the polymer dispersion index (PDI), a great PDI means that the pectin has a wide molecular weight distribution and contains a variety of molecules with different molecular weights. In the gel formation process, molecules of different molecular weights have different gelation behaviors, which can cause the gel formation time to be difficult to accurately control, and the quality of the gel is unstable, which may cause local excessive gelation or insufficient gelation, which may affect the texture stability and taste of the pectic gel, and at the same time increase the precipitation of water and oil; and if the PDI is too low, since the pectic gel is generally a complex formulation system and there will be certain interactions between various components, i.e., the PDI being too low means that the properties of the pectin are relatively fixed, and it may not be able to form a diversified synergy with other components, resulting in a decrease in the overall performance of the pectic gel, and an increase in the amount of precipitation of water and oil.

Thirdly, with respect to HG and RG-1, pectin is a complex polysaccharide present in the cell wall of plants, including homogalacturonan (HG), rhamnogalacturonan I (RG-1) and other structural domains. HG is the main structural domain of the pectin molecule, which is a linear homopolymer of α-1,4-galacturonic acid, known as the "smooth zone", reflecting the gel characteristics of the main body. RG-I has a highly branched feature, known as the "hair zone", and its side chain has a strong ability to bind water and a stable gel network structure. When the HG content is too low, the cross-linking points between the pectin molecules are reduced, and it is difficult to form a stable three-dimensional network structure. As a result, the strength of the gel is reduced, the structural stability is poor, it is more sensitive to changes in environmental factors such as temperature, pH value, and ionic strength, and the stability of the formed water-oil emulsion is worse. Therefore, the pectin gel after forming is not only poor in texture stability, but also prone to precipitation of water and oil. The HG content is controlled to > 30 mol %, effectively ensuring the high stability of the water-oil emulsion, so that the formed pectin gel has high strength, further ensuring that it has good texture stability, and reducing the amount of precipitation of water and oil. RG-1 has an abundance of neutral sugar side chains that increase the flexibility and spatial potential resistance of the pectin molecule, making the gel more resilient. When the content of RG-1 is too great, the elasticity of the gel will be further enhanced, and excessive elasticity may occur, resulting in the gel being prone to deformation when subjected to external forces, and this rebound force may destroy the original structure inside the gel, making the texture of the gel loose and reducing its stability. When multiple pectin gels with excessive elasticity are stacked or contacted with each other, they cannot maintain their respective shapes like texture-stable gels under the action of gravity or other external forces, but are easy to squeeze and deform each other and even fuse together, affecting the stability of the entire system. Therefore, the RG-1 is controlled to ≤ 35 mol %, which is conducive to avoiding the excessive elasticity of the pectin gel and affecting its overall texture stability, and reducing the amount of precipitation of water and oil.

It should be noted that, since pectin gel is a formula system, there are certain interactions between pectin, fat-soluble substances and other materials. It is by controlling the values of the weight average molar weight, polymer dispersion index (PDI), HG, and RG-1 of pectin so that the interactions of the components in the pectin gel reach a more balanced and stable state. In particular, it promotes the stable existence of fat-soluble substances in the mixing system, and further optimizes the stability of the mixed material solution (emulsion) and the texture stability of the gel after forming, reduces the amount of precipitation of water and oil of the pectin gel, and improves its storage stability and taste.

In some implementations, the weight average molar weight of the pectin is in a range of 8.0×10^4 to 5.0×10^5 Da, the polymer dispersion index thereof is in a range of 1 to 4; Further adjusting the weight average molar weight and polymer dispersion index of the pectin is more conducive to the overall stability of the mixed material solution, and thus is more conducive to obtaining a pectin gel with higher texture stability, less precipitation of water and oil, and better taste.

In some implementations, the molecular weight of the pectin is determined using a multi-angle laser light scatterometer.

In some implementations, in the structural domains of the pectin, HG = 40-90 mol% and RG-1 = 4-30 mol%. Further, if HG is too great, it is prone to dehydration and shrinkage due to the strong interaction force between molecules. This causes the moisture in the gel to be squeezed out, making the gel lower in volume and tighter in texture, affecting the appearance and quality of the product. If RG-1 is too low, the elasticity and toughness of the gel is significantly reduced, making the gel more fragile and prone to cracking. Therefore, further defining HG and RG-1 in a specific range is not only more conducive to obtaining a stable water-oil emulsion system, but also more conducive to obtaining a pectin gel that takes into account various properties such as texture stability, precipitation of water and oil, and taste.

In some implementations, HG = GalA-Rha, RG-1 = 2Rha + Ara + Gal; GalA is the molar percentage of galacturonic acid, Rha is the molar percentage of rhamnose, Ara is the molar percentage of arabinose, and Gal is the molar percentage of galactose, in which the molar percentages of HG and RG-1 are obtained as follows:
a. Determination of the Wt content of galacturonic acid, galactose, rhamnose, arabinose, xylose, glucose, and fructose, in 100 g of pectin by ion chromatography; b. Calculation of the corresponding molar number M and the sum thereof Mₛᵤₘ respectively according to the molar mass of the substance, and obtain the corresponding molar percentage according to mol % = M/Mₛᵤₘ × 100%. Relevant research shows that HG and RG-1 are calculated through the above formula, and the approximate value obtained is relatively close to the actual value. Therefore, the values of HG and RG-1 calculated by the above formula are relatively accurate.

In some implementations, the pectin gel further includes a sugar alcohol, in which the sugar alcohol includes a first sugar alcohol and a second sugar alcohol, the first sugar alcohol includes xylitol, and the second sugar alcohol includes at least one of maltitol and sorbitol. Alternatively, the sugar alcohol includes a first sugar alcohol, a second sugar alcohol, and a third sugar alcohol, the first sugar alcohol includes xylitol, the second sugar alcohol includes at least one of malitol and sorbitol, and the third sugar alcohol includes erythritol. Sugar alcohols can regulate the water activity and solids of the system, creating more favorable conditions for the formation of pectin gels. Regarding the types of sugar alcohols, sugar alcohols generally have a relatively high content in the pectin gel, so they also have a relatively important impact on the overall performance of the pectin gel. The present application further promotes the texture stability of the pectin gel by selecting specific sugar alcohol types to make it have a good interaction with pectin and fat-soluble substances, which reduces the precipitation of water and oil and improves the taste.

In some implementations, the sugar alcohol includes the first sugar alcohol and the second sugar alcohol, and a mass ratio between the first sugar alcohol and the second sugar alcohol is in a range of (1-30): (0.09-65); Alternatively, the sugar alcohol includes the first sugar alcohol, the second sugar alcohol, and the third sugar alcohol, and a mass ratio between the first sugar alcohol, the second sugar alcohol, and the third sugar alcohol is in a range of (1-30): (0.09-65): (0.1-10). Further controlling the type and ratio of sugar alcohol, the interaction of sugar alcohol with pectin, fat-soluble substances and other substances is more balanced, and does not affect the performance of various substances. Therefore, it is more conducive to maintaining the balance and stability of water-oil emulsions, thereby optimizing the texture stability of the pectin gel after forming, reducing the amount of precipitation of water and oil, and improving the taste of the pectin gel.

In some implementations, a mass ratio of the pectin, the sugar alcohol, and the fat-soluble substance, in terms of mass, is (0.5-5): (10-70): (5-50). Further defining the mass ratio of pectin, sugar alcohol and fat-soluble substances within a certain range is more conducive to the interaction of these three substances. At the same time, it is also more conducive to the interaction of these three substances with other substances in the pectin gel system, promoting the balance and stability of the water-oil emulsion, and further optimizing the texture stability of the formed pectin gel, reducing the amount of precipitation of water and oil, and improving the taste of the pectin gel.

In some implementations, the pectin gel further includes an emulsifier, and a mass ratio of the pectin, the sugar alcohol, the fat-soluble substance, and the emulsifier, in terms of mass, is (0.5-5): (10-70): (5-50): (0.02-1). Emulsifiers are the key factors for the stability of water-oil emulsions. Therefore, it is also necessary to add emulsifiers to stabilize the water-oil emulsions, the amount of emulsifiers is controlled within a certain range, which is more conducive to the stability of the water-oil emulsions, and furthermore, it is more conducive to obtaining pectin gels with high texture stability, low precipitation of water and oil, and good taste.

In some implementations, a mass percentage of the pectin in raw materials used in preparing the pectin gel is in a range of 1 to 4%.

In some implementations, an esterification degree of the pectin is >50%.

In some implementations, the fat-soluble substances include fat-soluble physiologically active substances including at least one of DHA algae oil, oil-soluble vitamins, evening primrose oil, arachidonic acid, linseed oil, gamma linolenic acid oil, capric triglyceride (MCT), safflower seed oil, milk thistle seed oil, acer truncatum bunge seed oil, walnut oil, fish oil, coenzyme Q10, rice bran fatty alkanol, pumpkin seed oil, borage oil, acetylsalicylic acid, fat-soluble statins, antibiotics, naproxen, antihistamines.

In some implementations, the pectin gel further includes an acidity regulator, and a mass ratio between the pectin and the acidity regulator is in a range of (0.5-5): (0.5-7.5).

In some implementations, the emulsifier includes at least one of modified starch, phospholipid, gum, acetylated pectin, cholesterol, lanolin, saponin, polysaccharide, and protein.

In some implementations, the modified starch includes an esterified starch; the phospholipid includes at least one of soybean phospholipid, modified phospholipid, enzymatic phospholipid; and the gum includes arabic gum.

In some implementations, the acidity regulator includes at least one of citric acid, sodium citrate, potassium citrate, malic acid and lactic acid.

In some implementations, the aforementioned pectic gel also contains metal ions, the metal ions include calcium ions. In some implementations, the calcium ions are provided by a calcium salt including at least one of calcium lactate, calcium citrate, tricalcium phosphate, calcium hydrogen phosphate, dairy mineral salt, calcium gluconate, calcium chloride.

According to a second aspect of the present application, provided is a preparation method of the pectin gel as described above, including the following steps: aqueous phase preparation: uniformly mixing the pectin, part of the sugar alcohol, and part of acidity regulator to obtain a gel premix; uniformly mixing water, the remaining sugar alcohol, and modified starch, adding the gel premix thereto, and heating to complete dissolution to obtain an aqueous phase; oil phase preparation: heating and mixing the fat-soluble substance until being uniform to obtain an oil phase; emulsification: adding the oil phase to the aqueous phase and mixing uniformly, performing high-speed shear emulsification at 70 to 95 °C to obtain an emulsion; blending: mixing the remaining acidity regulator and water, mixing at 70 to 80 °C until being completely dissolved, and adding to the emulsion for mixing for 5 to 10 minutes to obtain a material solution; forming: pouring the material solution on a blister, sealing, and standing still for 40 to 60 h to be formed stably.

In some implementations, during the preparation of the pectin gel described above, glycerin is added during the preparation of the aqueous phase, and the mass ratio between pectin and glycerin is in a range of (0.5-5): (0.1-8).

### BRIEF DESCRIPTION OF THE DRAWINGS

FIG. 1 shows a schematic diagram of the mixed material solution (water-oil emulsion) in examples 1 to 5 and comparative examples 1 to 2 in the present application after centrifugal stability testing.
FIG. 2 shows a schematic diagram of the mixed material solution (water-oil emulsion) in examples 1 to 5 and comparative examples 1 to 2 in the present application after thermal stability testing.

### DETAILED DESCRIPTION

For a better understanding of the solutions of the present application by those skilled in the art, the technical solutions in the examples of the present application are clearly and completely described and discussed below. Obviously, the examples described herein are only some of the examples of the present application but not all of them.

In the present application, the pectin used in the examples and comparative examples is selected from the standard commercialized materials. The material standards meet the requirements of GB 25533. The following table 1 shows the parameter information of each pectin with respect to Mw, PDI, HG, and RG-1.

**Table 1 Summary of parameter information about Mw, PDI, HG, RG-1 for each pectin**

| Material name | Weight average molar weight (Mw) | PDI (Polymer Dispersibility Index Mw/Mn) | HG (homogalacturonan) approximation, mol % | RG-1 (rhamnogalacturonan type I) approximation, mol % |
|---|---|---|---|---|
| Pectin A | 1.072×10^5±5.336% | 2.323±2.369% | 81±10% | 14±10% |
| Pectin B | 1.375×10^5±5.147% | 2.060±8.264% | 57±10% | 8±10% |
| Pectin C | 2.016×10^5±7.025% | 1.757±5.801% | 58±10% | 5±10% |
| Pectin D | 3.123×10^5±3.614% | 2.957±6.243% | 72±10% | 9±10% |
| Pectin E | 4.703×10^5±5.211% | 2.467±4.261% | 79±10% | 20±10% |
| Pectin F | 7.328×10^4±3.121% | 1.231±5.352% | 46±10% | 5±10% |
| Pectin G | 4.534×10^4±2.326% | 2.127±9.215% | 45±10% | 3±10% |
| Pectin H | 6.721×10^5±6.132% | 1.757±7.025% | 80±10% | 19±10% |
| Pectin I | 3.879×10^5±4.366% | 6.537±5.645% | 55±10% | 4±10% |
| Pectin J | 1.825×10^5±5.137% | 2.160±5.264% | 55±10% | 10±10% |
| Pectin K | 1.360×10^5±5.007% | 2.012±3.264% | 80±10% | 10±10% |
| Pectin L | 4.263×10^5±6.201% | 2.837±6.321% | 80±10% | 20±10% |
| Pectin M | 1.120×10^5±4.756% | 1.931±4.830% | 25±10% | 2±10% |

### Examples and comparative examples

1. The effects of Mw, and/or PDI of pectin on water-oil emulsions and formed pectin gels were investigated using Mw, and/or PDI of pectin as variables.

A total of six examples and three comparative examples are set out above, and the formulations of each example and the comparative examples are shown in Table 2.

**Table 2 Formulations of examples 1 to 6 and comparative examples 1 to 3**

| Components | | | Exa mple 1 | Exa mple 2 | Exa mple 3 | Exa mple 4 | Exa mple 5 | Exa mple 6 | Comp arativ e exam ple 1 | Comp arativ e exam ple 2 | Comp arativ e exam ple 3 |
|---|---|---|---|---|---|---|---|---|---|---|---|
| Aque ous phase | Pecti n | Pect in A | 2% | / | / | / | / | / | / | / | / |
| | | Pect in B | / | 2% | / | / | / | / | / | / | / |
| | | Pect in C | / | / | 2% | / | / | / | / | / | / |
| | | Pect in D | / | / | / | 2% | / | / | / | / | / |
| | | Pect in E | / | / | / | / | 2% | / | / | / | / |
| | | Pect in F | / | / | / | / | / | 2% | / | / | / |
| | | Pect in G | / | / | / | / | / | / | 2% | | / |
| | | Pect in H | / | / | / | / | / | / | / | 2% | |
| | | Pect in I | / | / | / | / | / | / | / | / | 2% |
| | emuls ifier | mod ifie d star ch | 0.07 % | 0.07 % | 0.07 % | 0.07 % | 0.07 % | 0.07 % | 0.07% | 0.07% | 0.07% |
| | sugar alcoh ol | xyli tol | 15% | 15% | 15% | 15% | 15% | 15% | 15% | 15% | 15% |
| | | malt itol solu tion | 31% | 31% | 31% | 31% | 31% | 31% | 31% | 31% | 31% |
| | acidit y regul ator | sodi um citra te | 0.4% | 0.4% | 0.4% | 0.4% | 0.4% | 0.4% | 0.4% | 0.4% | 0.4% |
| | | citri c acid | 0.2% | 0.2% | 0.2% | 0.2% | 0.2% | 0.2% | 0.2% | 0.2% | 0.2% |
| | / | glyc erin | 5% | 5% | 5% | 5% | 5% | 5% | 5% | 5% | 5% |
| | solve nt | puri fied wat er | remn ant | remn ant | remn ant | remn ant | remn ant | remn ant | remna nt | remna nt | remna nt |
| oil phase | Fat-solub le comp onent s | DH A alga l oil | 25% | 25% | 25% | 25% | 25% | 25% | 25% | 25% | 25% |
| Blend ing comp onent s | acidit y regul ator | citri c acid | 0.7% | 0.7% | 0.7% | 0.7% | 0.7% | 0.7% | 0.7% | 0.7% | 0.7% |
| | solve nt | puri fied wat er | appr opria te amou nt | appr opria te amou nt | appr opria te amou nt | appr opria te amou nt | appr opria te amou nt | appr opria te amou nt | appro priate amou nt | appro priate amou nt | appro priate amou nt |

2. The effects of the sugar alcohol type of pectin as well as the ratio thereof on water-oil emulsion and formed pectin gel are investigated using the sugar alcohol type as well as the ratio thereof as variables.

A total of fifteen examples are set out above, and the formulations of each example are shown in Table 3-1, Table 3-2, and Table 3-3.

**Table 3-1 Formulations for Examples 7 to 12**

| Components | | | Examp le 7 | Examp le 8 | Examp le 9 | Examp le 10 | Examp le 11 | Examp le 12 |
|---|---|---|---|---|---|---|---|---|
| Aqueo us phase | Pectin | Pectin D | 2% | 2% | 2% | 2% | 2% | 2% |
| | Emulsifi er | modified starch | 0.07% | 0.07% | 0.07% | 0.07% | 0.07% | 0.07% |
| | sugar alcohol | xylitol | 42% | 42% | 0.75% | 0.75% | 0.65% | 0.65% |
| | | sorbitol solution | / | 4.2% | / | 45% | / | 39% |
| | | erythritol | / | / | / | / | 6.5% | 6.5% |
| | | maltitol solution | 4.2% | / | 45% | / | 39% | / |
| | | xylitol: maltitol solution/sorbi tol solution: erythritol | 1:0.1:0 | 1:0.1:0 | 1:60:0 | 1:60:0 | 1:60:10 | 1:60:10 |
| | acidity regulator | sodium citrate | 0.4% | 0.4% | 0.4% | 0.4% | 0.4% | 0.4% |
| | | citric acid | 0.2% | 0.2% | 0.2% | 0.2% | 0.2% | 0.2% |
| | / | glycerin | 5% | 5% | 5% | 5% | 5% | 5% |
| | solvent | purified water | remnan t | remnan t | remnan t | remnan t | remnan t | remnan t |
| oil phase | Fat-soluble compone nts | DHA algal oil | 25% | 25% | 25% | 25% | 25% | 25% |
| Blending compone nts | acidity regulator | citric acid | 0.7% | 0.7% | 0.7% | 0.7% | 0.7% | 0.7% |
| | solvent | purifi ed water | appropri ate amount | appropri ate amount | appropri ate amount | appropri ate amount | appropri ate amount | appropri ate amount |

**Table 3-2 Formulations for Examples 13 to 18**

| Components | | | Exampl e 13 | Exampl e 14 | Exampl e 15 | Exampl e 16 | Exampl e 17 | Exampl e 18 |
|---|---|---|---|---|---|---|---|---|
| | | Pectin D | 2% | 2% | 2% | 2% | 2% | 2% |
| | Emulsif ier | Modified starch | 0.07% | 0.07% | 0.07% | 0.07% | 0.07% | 0.07% |
| | sugar alcohol | xylitol | 46% | 46% | 15% | 15% | 14% | 14% |
| | | sorbitol solution | / | 0.15% | / | 31% | / | 28% |
| | | erythritol | / | / | / | / | 4.6% | 4.6% |
| | | maltitol solution | 0.15% | / | 31% | / | 28% | / |
| | | xylitol: maltitol solution/so rbitol solution: erythritol | 30:0.09 8:0 | 30:0.09 8:0 | 30:62:0 | 30:62:0 | 30:60:1 0 | 30:60:1 0 |
| | acidity regulato r | sodium citrate | 0.4% | 0.4% | 0.4% | 0.4% | 0.4% | 0.4% |
| | | citric acid | 0.2% | 0.2% | 0.2% | 0.2% | 0.2% | 0.2% |
| | / | glycerin | 5% | 5% | 5% | 5% | 5% | 5% |
| | solvent | purified water | remnan t | remnan t | remnan t | remnan t | remnan t | remnan t |
| oil phase | Fat-soluble compon ents | DHA algal oil | 25% | 25% | 25% | 25% | 25% | 25% |
| Blendin g compon ents | acidity regulato r | citric acid | 0.7% | 0.7% | 0.7% | 0.7% | 0.7% | 0.7% |
| | solvent | purified water | appropr iate amount | appropr iate amount | appropr iate amount | appropr iate amount | appropr iate amount | appropr iate amount |

**Table 3-3 Formulations for Examples 19 to 21**

| Components | | | Example 19 | Example 20 | Example 21 |
|---|---|---|---|---|---|
| Aqueous phase | Pectin | Pectin D | 2% | 2% | 2% |
| | Emulsifier | Modified starch | 0.07% | 0.07% | 0.07% |
| | sugar alcohol | xylitol | 46% | / | / |
| | | sorbitol solution | / | / | / |
| | | erythritol | / | / | 46% |
| | | maltitol solution | / | 46% | / |
| | | xylitol: maltitol solution/sorbitol solution: erythritol | 1:0:0 | 0:1:0 | 0:0:1 |
| | acidity regulator | sodium citrate | 0.4% | 0.4% | 0.4% |
| | | citric acid | 0.2% | 0.2% | 0.2% |
| | / | glycerin | 5% | 5% | 5% |
| | solvent | purified water | remnant | remnant | remnant |
| oil phase | Fat-soluble components | DHA algal oil | 25% | 25% | 25% |
| Blending components | acidity regulator | citric acid | 0.7% | 0.7% | 0.7% |
| | solvent | purified water | appropriate amount | appropriate amount | appropriate amount |

3. The effects of HG, and/or RG-1 of pectin on water-oil emulsions and formed pectin gels were investigated using HG, and/or RG-1 of pectin as variables.

A total of three examples and one comparative example are set out above, and the formulations of each example and the comparative example are shown in Table 4.

**Table 4 Formulations of examples 22 to 24 and comparative example 4**

| Components | | | Example 22 | Example 23 | Example 24 | Comparative example 4 |
|---|---|---|---|---|---|---|
| Aqueous phase | Pectin | Pectin J | 2% | / | / | / |
| | | Pectin K | / | 2% | / | / |
| | | Pectin L | / | / | 2% | / |
| | | Pectin M | / | / | / | 2% |
| | Emulsifier | Modified starch | 0.07% | 0.07% | 0.07% | 0.07% |
| | sugar alcohol | xylitol | 15% | 15% | 15% | 15% |
| | | maltitol solution | 31% | 31% | 31% | 31% |
| | acidity regulator | sodium citrate | 0.4% | 0.4% | 0.4% | 0.4% |
| | | citric acid | 0.2% | 0.2% | 0.2% | 0.2% |
| | / | glycerin | 5% | 5% | 5% | 5% |
| | solvent | purified water | remnant | remnant | remnant | remnant |
| oil phase | Fat-soluble components | DHA algal oil | 25% | 25% | 25% | 25% |
| Blending components | acidity regulator | citric acid | 0.7% | 0.7% | 0.7% | 0.7% |
| | solvent | purified water | appropriate amount | appropriate amount | appropriate amount | appropriate amount |

### Test example

### 1. Experimental construction method

(1) Preparation of pectin gel: pectin gels were respectively prepared according to formulations in all the above examples and the comparative examples. The specific preparation steps are as follows:
   aqueous phase preparation: pectin, a part of sugar alcohol, a part of acidity regulator (sodium citrate, citric acid) were mixed uniformly to obtain a gel premix; water, remaining sugar alcohol (if any, "if any" means sugar alcohol may also be all added directly when preparing the gel premix), modified starch, glycerin were mixed evenly, and it was heated to complete dissolution after adding the gel premix, so as to obtain an aqueous phase;
   oil phase preparation: the fat-soluble substance (DHA algal oil) was heated to 90 °C (if there are multiple types of fat-soluble substances, they need to be heated until they are mixed evenly, and then insulated), and insulated to obtain an oil phase;
   emulsification: the oil phase was added to the aqueous phase and mixed uniformly, and high-speed shear emulsification was performed at 90 °C to obtain an emulsion; blending: the remaining acidity regulator (citric acid) and water were mixed at 70 to 80 °C until being completely dissolved, and then added to the emulsion for mixing for 5 to 10 minutes to obtain a material solution;
   forming: the material solution was poured on a blister, sealed, and stood still for 48 hours to be formed stably.
(2) Performance test
   ①Emulsification stability measurement
      a. Centrifugal stability
         10 g of the unformed pectin gel (material solution) after blending was taken and placed in a centrifuge tube, balanced in a water bath at 75 °C for 30 minutes, and centrifuged at 3000 rpm for 10 minutes. The delamination of the emulsion gel was observed and recorded.
      b. Thermal stability
         10 g of the unformed pectin gel (material solution) after blending was taken and placed in a centrifuge tube, heated in a water bath at 90 °C for 1h, taken out, and then centrifuged at 3000 rpm for 10 minutes. The delamination of the emulsion gel was observed and recorded.
      c. Freeze-thaw stability
         10 g of the unformed pectin gel (material solution) after blending was taken and placed in a centrifuge tube, sealed with an aluminum compound bag, and stored in a refrigerator at -20 °C for 24 hours. It was taken out and defrosted at room temperature for 24 hours, then centrifuged at 3000 rpm for 10 minutes. The delamination of the emulsion gel was observed and recorded.
   ②Degree of water discharge test
      10 g of stable formed pectin gel was placed in a standard open container, then placed in a constant temperature drying oven at 25 ± 2 °C, 20 - 30% RH, and dried for 24 hours, 48 hours, respectively, with drying loss/% = (M1-M2)/M1, and drying balance = |(M1-M3)/M1-drying loss|, which was repeated three times, and the average value was taken, where M1 indicates the weight before drying, M2 indicates the weight after drying for 24h, and M3 indicates the weight after drying for 48h.
   ③Texture test
      The SMS TA XT plus texture meter was employed to test by means of the TPA test mode. Pre-test speed: 1 mm/s, test speed: 3 mm/s, post-test speed: 3 mm/s, triggering force: 5 g, target mode: 50% of deformation amount. The pectin gel was controlled to be the same size and shape, round pie in shape, 1.5g/granule, and each group of samples were tested six times in parallel.
   ④Disintegration time
      Test was performed according to USP < 2040 > (medium water).
(3) Assessment standards

The relevant performances of the emulsion (material solution) and the pectin gel after forming in the examples and comparative examples were assessed according to the assessment standards in Table 5 below.

**Table 5 Assessment standards**

| Evaluation indicators | | Weight | Score | | | |
|---|---|---|---|---|---|---|
| | | | 10 | 7 | 4 | 0 |
| Emulsification stability | Centrifugal stability | 20% | Non-stratified | Stratified and H_{lower} ≤ 1/5 | H_{lower} > 1/5 | Unable to prepare a homogeneous emulsion |
| | Thermal stability | 20% | Non-stratified | Stratified and H_{lower} ≤ 1/3 | H_{lower} > 1/3 | |
| | Freeze-thaw stability | 10% | Non-stratified | Stratified and H_{lower} ≤ 1/5 | H_{lower} > 1/5 | |
| Degree of water discharge test | Loss of weight on drying over 24h | 35% | <2% | 2%≤x≤4% | >4% | |
| | Drying balance | 15% | <2% | 2%≤x<4% | ≥4% | |
| Whether there is crystal precipitation | | 50% | None | / | / | a Yes b Unable to prepare uniform emulsion |
| Texture testing | Hardness | 34% | ≥1500g | 500g to 1500g | <500g | a Unable to prepare a uniform emulsion b Able to prepare a uniform emulsion, but unable to be formed |
| | Adhesiveness | 33% | ≥-30 | -30≤x≤-50 | <-50 | |
| | Resilience | 33% | ≥0.30 | 0.24 to 0.30 | <0.24 | |

### 2. Test results

The relevant performance assessment results of the emulsion (material solution) and the pectin gel after forming in the examples and comparative examples are shown in Tables 6-1 and 6-2.

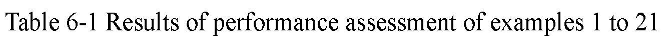

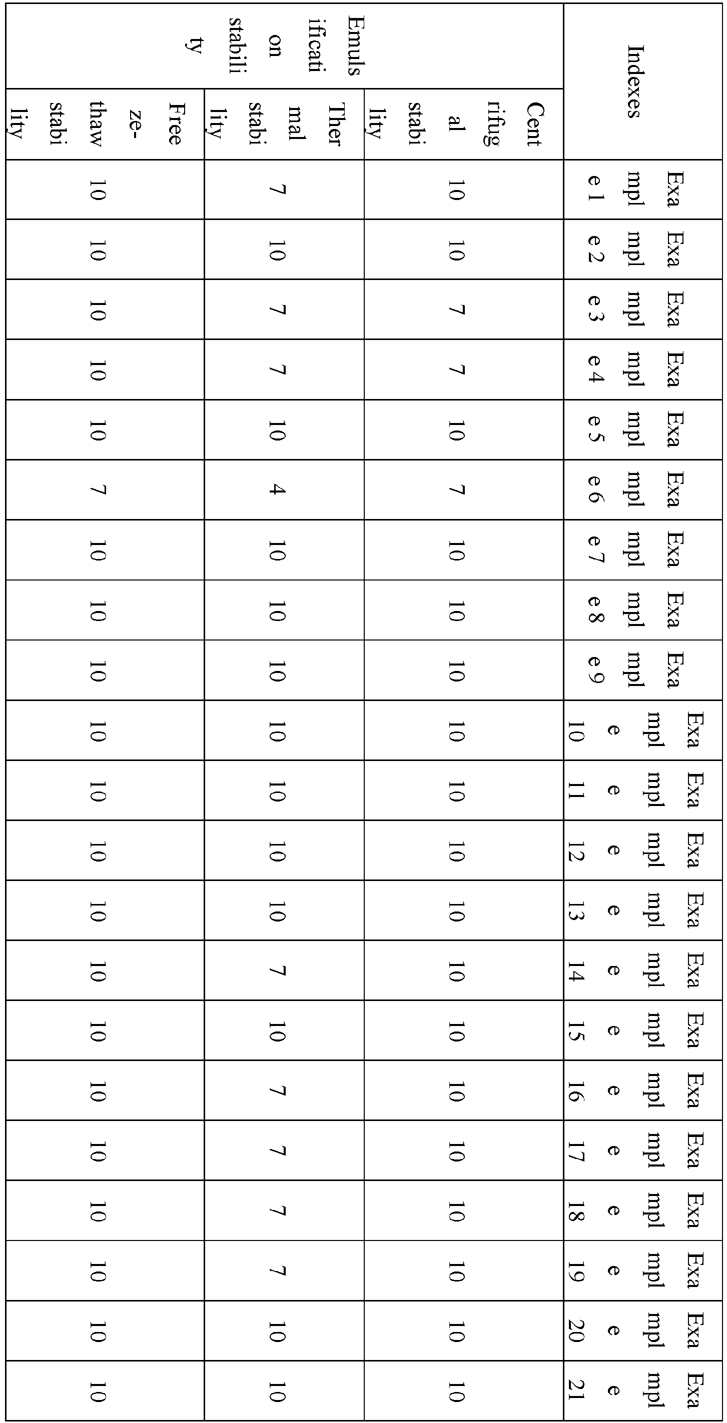

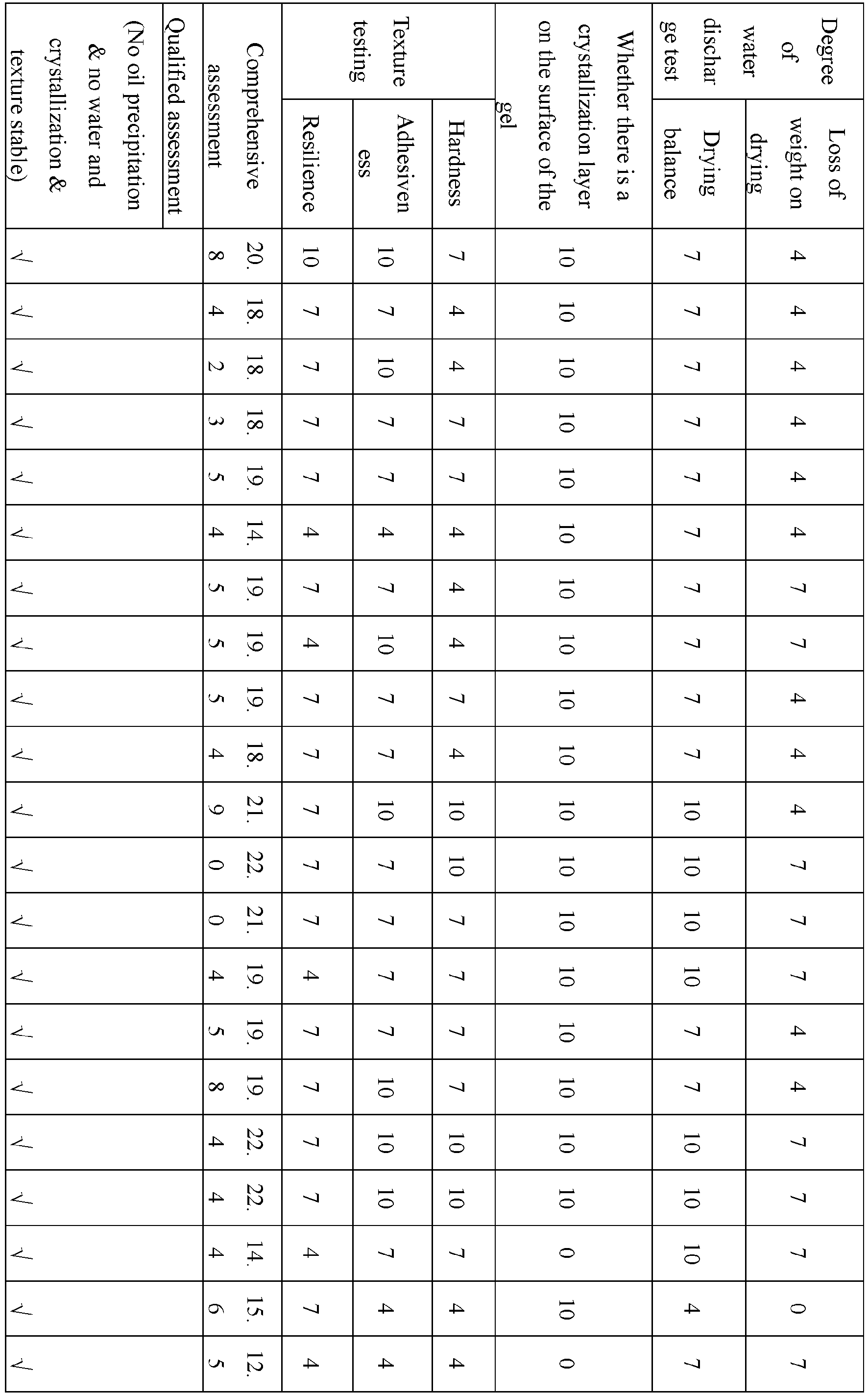

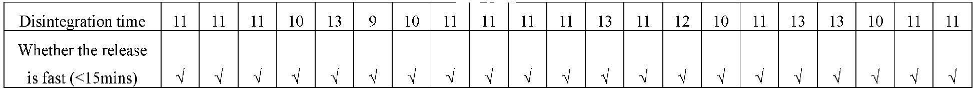

**Table 6-2 Results of performance assessment of examples 22 to 24 and comparative examples 1 to 4**

| Indexes | | Example 22 | Example 23 | Example 24 | Comparative example 1 | Comparative example 2 | Comparative example 3 | Comparative example 4 |
|---|---|---|---|---|---|---|---|---|
| Emulsion Stability | Centrifugal stability | 10 | 10 | 10 | 4 | 0 | 10 | 4 |
| | Thermal stability | 10 | 7 | 10 | 4 | 0 | 7 | 4 |
| | Freeze-thaw stability | 10 | 10 | 10 | 7 | 0 | 10 | 7 |
| Degree of water discharge test | Loss of weight on drying | 4 | 4 | 4 | 4 | 0 | 4 | 4 |
| | Drying balance | 7 | 7 | 7 | 7 | 0 | 4 | 7 |
| Whether there is a crystallization layer on the surface of the gel | | 10 | 10 | 10 | 10 | 0 | 10 | 10 |
| Texture testing | Hardness | 4 | 7 | 7 | 0 | 0 | 0 | 0 |
| | Adhesiveness | 7 | 10 | 7 | 0 | 0 | 0 | 0 |
| | Resilience | 7 | 10 | 7 | 0 | 0 | 0 | 0 |
| Comprehensive assessment | | 18.4 | 20.8 | 19.5 | 9.8 | 0 | 11.4 | 9.8 |
| Qualified assessment (no oil precipitation & no water and crystallization & texture stable) | | √ | √ | √ | × | × | × | × |
| Disintegration time | 11 | 11 | 13 | N/A | N/A | N/A | N/A | |
| Whether the release is fast (<15mins) | √ | √ | √ | N/A | N/A | N/A | N/A | |

As shown in tables 6-1 and 6-2 that, in the present application, by controlling the weight average molar weight of the pectin, the polymer dispersion index (PDI), and the type of defined sugar alcohol, the stability of the material solution (emulsion) before forming was effectively improved, which improves the pectin gel texture stability after forming, effectively reduces the amount of precipitation of water and oil in the pectin gel, greatly improves the storage stability of the pectin gel, and improves the taste of the pectin gel. Please refer specifically to the performance data in examples 1 to 24.

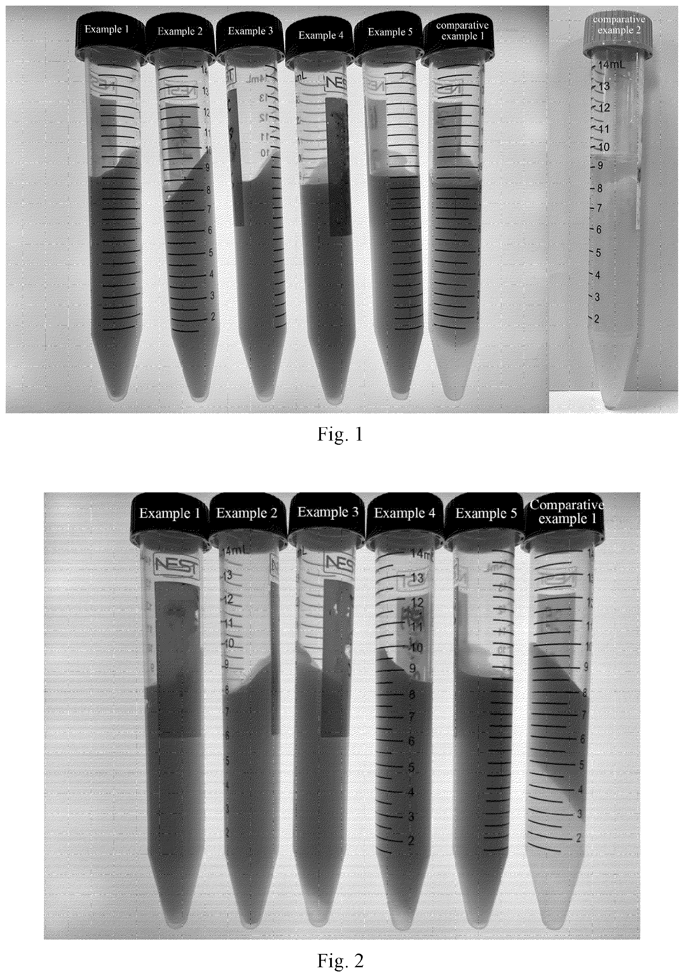

The weight average molar weight Mw of pectin in comparative examples 1 and 2 was too low and too high, respectively, which was not conducive to the formation of a stable emulsion in the mixed material solution (emulsion), and was not conducive to the stability of the pectin gel texture after forming, and even affected the formation of the pectin gel. Therefore, the emulsion or oil-water delamination phenomenon after the centrifugation of comparative examples 1 and 2 was serious. Refer to the centrifuge tube pictures of comparative examples 1 and 2 in Fig. 1. The centrifuged emulsion or oil-water had a certain degree of delamination or serious delamination. Compared with comparative examples 1 and 2, with further reference to Fig. 1, it should be noted that the weight average molar weight Mw of examples 1 to 5 were within a suitable range, so that their emulsions still had good stability after centrifugation, and were not obviously delaminated. However, a relatively stable emulsion was not able to be formed in the comparative examples 1 and 2, leading to inability to form into pectin gels or reduced textural stability of the formed pectin gels, the amount of precipitation of water and oil was high, the comprehensive evaluation score was low, and the pectin gel was unqualified. With further reference to Fig. 2, it can also be seen that the thermal stability of the emulsion in comparative example 1 was worse than that in examples 1 to 5, which also indicated that the overall stability of the emulsion in comparative example 1 was poor, which was not conducive to the preparation of pectin gels with high texture stability and low amount of precipitation of water and oil.

The polymer dispersion index of pectin in comparative example 3 was too high, the molecular weight distribution of pectin was too wide, the stability of the emulsion was decreased, and the quality of the gel was also unstable. It is prone to localized excessive or insufficient gelation, which in turn affects the texture stability of the pectin gel. As a result, the comprehensive evaluation score of the final pectin gel was also low, and it was prone to precipitation of water and oil, and the pectin gel was also unqualified. In pectin of comparative example 4, the HG was relatively low, and the RG-1 was also relatively low, leading to a decrease in the stability of the emulsion in comparative example 4, and the texture stability of the gel after forming was relatively poor, and the amount of precipitation of water and oil was also relatively high, the comprehensive evaluation score was also low, and the pectin gel was unqualified.

Further comparing examples 1 to 5 and example 6, the weight average molar weight Mw of the pectin in examples 1 to 5 was in the further preferred range of 8.0×10^4 to 5.0×10^5 Da, while the weight average molar weight Mw of the pectin in example 6 was not in this range, resulting in a lower comprehensive evaluation score of the pectin gel in example 6, which indicated that further controlling the weight average molar weight of the pectin was more conducive to obtaining a pectin gel with higher texture stability, less precipitation of water and oil, and better taste. Similarly, the polymer dispersion index PDI of pectin was similar, which is not be illustrated here.

In view of examples 7 to 21, it should be noted that, during the preparation of the pectin gel, when the types of sugar alcohols included xylitol and maltitol (or sorbitol) and the ratio of each sugar alcohol was within a certain range, the prepared emulsion was relatively stable, the pectin gel structure stability after forming was also high, the water retention and moisture resistance coordination were good, the comprehensive evaluation score was high, and the pectin gel was qualified. Further, it can be found that, when the sugar alcohol includes three types thereof, the comprehensive evaluation score is higher. It indicates that, when the sugar alcohol includes three types thereof and is in a certain proportion range, it is more conducive to obtaining a pectin gel with high texture stability, low precipitation of oil and water, and good taste.

The above examples are only used to illustrate the technical solution of the present application rather than to limit the protection scope of the present application. Although the present application has been described in detail with reference to the above examples, a person of ordinary skill in the art should be aware that modifications or equivalent substitutions may be carried out to the technical solution of the present application, these modifications or substitutions fall within the protection scope of the present application.

## Claims

1. A pectin gel, comprising a pectin and a fat-soluble substance, wherein a weight average molar weight of the pectin is in a range of 5.0×10^4 to 6.0×10^5 Da and a polymer dispersion index thereof is in a range of 1 to 6; in structural domains of the pectin, HG > 30 mol% and RG-1 ≤ 35 mol%; and HG is homogalacturonan and RG-1 is rhamnogalacturonan type I.

2. The pectin gel according to claim 1, wherein the weight average molar weight of the pectin is in a range of 8.0×10^4 to 5.0×10^5 Da, the polymer dispersion index thereof is in a range of 1 to 4.

3. The pectin gel according to claim 1, wherein, in structural domains of the pectin, HG = 40-90 mol% and RG-1 = 4-30 mol%.

4. The pectin gel according to claim 1, further comprising a sugar alcohol, wherein the sugar alcohol comprises a first sugar alcohol and a second sugar alcohol, the first sugar alcohol comprises xylitol, and the second sugar alcohol comprises at least one of maltitol and sorbitol.

5. The pectin gel according to claim 1, further comprising a sugar alcohol, wherein the sugar alcohol comprises a first sugar alcohol, a second sugar alcohol, and a third sugar alcohol, the first sugar alcohol comprises xylitol, the second sugar alcohol comprises at least one of malitol and sorbitol, and the third sugar alcohol comprises erythritol.

6. The pectin gel according to claim 4, wherein a mass ratio between the first sugar alcohol and the second sugar alcohol is in a range of (1-30): (0.09-65).

7. The pectin gel according to claim 5, wherein a mass ratio between the first sugar alcohol, the second sugar alcohol, and the third sugar alcohol is in a range of (1-30): (0.09-65): (0.1-10).

8. The pectin gel according to claim 4, wherein a mass ratio of the pectin, the sugar alcohol, and the fat-soluble substance, in terms of mass, is (0.5-5): (10-70): (5-50).

9. The pectin gel according to claim 5, wherein a mass ratio of the pectin, the sugar alcohol, and the fat-soluble substance, in terms of mass, is (0.5-5): (10-70): (5-50).

10. The pectin gel according to claim 8, further comprising an emulsifier, a mass ratio of the pectin, the sugar alcohol, the fat-soluble substance, and the emulsifier, in terms of mass, is (0.5-5): (10-70): (5-50): (0.02-1).

11. The pectin gel according to claim 8, further comprising an emulsifier, a mass ratio of the pectin, the sugar alcohol, the fat-soluble substance, and the emulsifier, in terms of mass, is (0.5-5): (10-70): (5-50): (0.02-1).

12. The pectin gel according to claim 1, wherein a mass percentage of the pectin in raw materials used in preparing the pectin gel is in a range of 1 to 4%.

13. The pectin gel according to claim 10, further comprising an acidity regulator, a mass ratio between the pectin and the acidity regulator is in a range of (0.5-5): (0.5-7.5).

14. A preparation method of the pectin gel according to any one of claims 1-13, comprising following steps:
aqueous phase preparation: uniformly mixing the pectin, part of a sugar alcohol, and part of acidity regulator to obtain a gel premix; uniformly mixing water, the remaining sugar alcohol, and modified starch, adding the gel premix thereto, and heating to complete dissolution to obtain an aqueous phase;
oil phase preparation: heating and mixing the fat-soluble substance until being uniform to obtain an oil phase;
emulsification: adding the oil phase to the aqueous phase and mixing uniformly, and performing high-speed shear emulsification at 70 to 95 °C to obtain an emulsion;
blending: mixing the remaining acidity regulator and water at 70 to 80 °C until being completely dissolved, and then adding to the emulsion for mixing for 5 to 10 minutes to obtain a material solution; and
forming: pouring the material solution on a blister, sealing, and standing still for 40 to 60 hours to be formed stably.
